Europäisches Patentamt

European Patent Office

Office européen des brevets

(19)

(11) Numéro de publication: **0 004 804**

**B1**

(12)

# FASCICULE DE BREVET EUROPEEN

(21) Numéro de dépôt: **79400171.9**

(22) Date de dépôt: **16.03.79**

(51) Int. Cl.³: **C 07 C 11/02, C 07 C 2/14**

(54) **Nouveau procédé de dimérisation d'oléfines en C8 - C20 et dimères d'oléfines ainsi obtenus**

(30) Priorité: **28.03.70 FR 7808852**

(43) Date de publication de la demande:
**17.10.79 Bulletin 79/21**

(45) Mention de la délivrance du brevet:
**29.10.80 Bulletin 80/22**

(84) Etats contractants désignés:
**DE GB NL**

(56) Documents cités:
**US - A - 3 953 538**

(73) Titulaire: **RHONE-POULENC INDUSTRIES
22, avenue Montaigne
F - 75008 Paris (FR)**

(72) Inventeur: **Desbois, Michel
570 Chemin du Bois
F - 69140 Rillieux (FR)**

(74) Mandataire: **Fabre, Madeleine-France et al
RHONE POULENC service Brevets Chimie et
Polymères B.P. 753
F - 75360 Paris Cedex 08 (FR)**

Courier Press, Leamington Spa, England.

Nouveau procédé de dimérisation d'oléfines en C$_8$—C$_{20}$, et dimères d'oléfines ainsi obtenus

La présente invention a pour objet un nouveau procédé de dimérisation d'oléfines en C$_8$—C$_{20}$ en présence d'acide fluorhydrique, ainsi que les dimères d'oléfines ainsi obtenus.

Il a été proposé (brevet américain n° 2 830 106) de dimériser les oléfines en C$_6$—C$_{15}$, en particulier les $\alpha$ oléfines, en présence d'un catalyseur à base d'alumine activée contenant de 0,5 à 3% en poids d'acide fluorhydrique Un tel procédé présente de nombreux inconvénients, à savoir de n'obtenir qu'un faible rendement en dimères, ainsi qu'une grande complexité dans la préparation du catalyseur.

Il a également été proposé de transformer des oléfines en C$_2$—C$_{12}$ en polymères en C$_{12}$—C$_{40}$ par mise en contact desdites oléfines avec de l'acide fluorhydrique liquide à une température comprise entre 10 et 60°C; tel que décrit ce procédé ne permet pas de préparer des dimères d'oléfines avec un rendement élevé.

La demanderesse a trouvé que seules des conditions bien particuliéres de mise en oeuvre du procédé de polymérisation en présence d'acide fluorhydrique liquide permettaient d'obtenir des dimères avec un rendement élevé.

Le procédé faisant l'object de l'invention est un procédé de dimérisation d'oléfines en C$_8$—C$_{20}$ par mise en contact desdites oléfines avec de l'acide fluorhydrique liquide à une température comprise entre —20 et 60°C, caractérisé:

en ce que ladite opération de mise en contact est réalisée pendant 1 à 30 minutes avec un rapport molaire acide fluorhydrique/oléfine compris entre 1/1 et 50/1;

en ce que après l'opération de mise en contact l'on sépare quasi instantanément du milieu dimérisation l'acide fluorhydrique présent;

en ce que l'on sépare dudit milieu les composés fluorés par défluoration

et en ce que l'on récupère les dimères d'oléfines obtenus.

Le procédé de l'invention peut être réalisé en continu ou en discontinu; une réalisation en continu est toutefois préférée.

Le procédé objet de l'invention est particulièrement intéressant pour la dimérisation des $\alpha$ oléfines en C$_{10}$—C$_{16}$ telles que le décène-1, le dodécène-1, le tétradécène-1, l'hexadécène-1.

L'étape de mise en contact est réalisée sous agitation rapide par exemple à l'aide d'un agitateur du type turbine ou à vis sans fin, de préférence sous une pression de 5 à 10 bars.

L'étape de séparation quasi instantanée de l'acide fluorhydrique aussitôt après l'étape de mise en contact peut par exemple être réalisée par une opération de distillation rapide du type "flash" du milieu de dimérisation pour éliminer sous forme vapeur l'acide fluorhydrique liquide libre, ou par neutralisation du milieu de dimérisation par les agents classiques de neutralisation de l'acide fluorhydrique, tels que la potasse en solution aqueuse.

L'étape de défluoration peut être réalisée par exemple par stripping, c'est-à-dire par extraction à une température supérieure à 200°C à l'aide d'un hydrocarbure aliphatique saturé ou aromatique inerte vis à vis de l'acide fluorhydrique, du type dodécane, ditertbutylbenzène, xylène . . . . ; cette étape peut également être réalisée par traitement à l'alumine à une température comprise entre 50 et 250°C. On obtient ainsi un mélange d'oligomères contenant une majeure partie de dimères et éventuellement un peu d'oléfine non oligomérisée qui peut être éliminée par simple distillation.

La séparation des dimères du mélange d'oligomères peut être réalisée par distillation.

Le procédé de dimérisation d'oléfines en C$_8$—C$_{20}$ objet de l'invention, permet d'obtenir des dimères d'oléfines avec des rendements élevés; ainsi ledit procédé réalisé avec un temps de contact de l'acide fluorhydrique et de l'oléfine compris entre 5 et 20 minutes permet d'obtenir des dimères d'oléfines avec un rendement supérieur à 60%.

La présente invention a également pour object les dimères d'oléfines ainsi obtenus.

Ces produits ont les applications classiques des oléfines à chaine longue.

Les exemples suivants sont donnés à titre indicatif et ne peuvent être considérés comme une limite du domaine et de l'esprit de l'invention.

*Exemple n° 1*

Dans un réacteur double enveloppe de 400 cm3 fortement agité et dont la température est contrôlée et régulée à 20°C on introduit par l'intermédiaire de pompes doseuses du tétradécène-1 à la cadence de 1,4 litre/heure et l'acide fluorhydrique anhydre à la cadence de 1 litre/heure, ce qui correspond à un rapport molaire Acide/oléfine de 9 et un temps de contact de 10 minutes.

Le mélange réactionnel qui sort en continu de ce réacteur est porté durant un temps très bref (quelques secondes) à une température de 100 à 140°C de façon à éliminer la majeure partie de l'HF sous forme gazeuse.

Les composés ainsi traités sont alors recueillis dans un pot contenant de la potasse aqueuse à 10% destinée à piéger les dernières traces d'acide libre. Après décantation et séchage sur chlorure de calcium du mélange précédent, on obtient le composé brut non défluoré dont la composition déterminée par analyse chromatographique (perméation de gel) et mesure du taux de fluor, est la suivante:

| | % poids |
|---|---|
| Oléfine non oligomérisée | 2,7 |
| Fluorotétradécane | 1,3 |
| Dimère d'oléfine | 52,0 |
| Fluorure du dimère d'olèfine $C_{14}$ | 26,0 |
| composés plus lourds (trimères, Tétramères et fluorures correspondants) | 18,0 |

Ce mélange est alors soumis à une défluoration par stripping au dodécane à 215°C pour donner un composé défluoré dont la composition est la suivante:

| | % poids |
|---|---|
| Oléfine non oligomérisée | 4 |
| Dimère d'oléfine | 81 |
| Produits plus lourds | 15 |

*Exemple 2*

On opère selon la méthode décrite à l'exemple 1 avec les conditions suivantes:

| | |
|---|---|
| Nature de l'oléfine | dodécène-1 |
| Température | 0°C |
| Cadence d'introduction de l'oléfine | 1,28 l/heure |
| Cadence d'introduction de l'acide | 3,52 l/heure |
| Rapport molaire acide/oléfine | 30 |
| Temps de contact | 5 minutes |

On obtient un produit fini défluoré de compositio

| | % poids |
|---|---|
| Oléfine non oligomérisée | 2 |
| Dimère d'oléfine | 65 |
| Produits plus lourds | 33 |

*Exemple 3*

On opère selon la méthode décrite à l'exemple 1 avec les conditions suivantes:

| | |
|---|---|
| Nature de l'oléfine | hexadécène-1 |
| Température | 40°C |

| | |
|---|---|
| Cadence d'introduction de l'oléfine | 0,99 l/heure |
| Cadence d'introduction de l'acide | 0,21 l/heure |
| Rapport molaire acide/oléfine | 3 |
| Temps de contact | 20 minutes |

On obtient un produit fini défluoré dont la composition est la suivante:

| | % poids |
|---|---|
| Oléfine non oligomérisée | 7 |
| Dimère d'oléfine | 74 |
| Produits plus lourds | 19 |

*Exemple 4*

On opère selon la méthode décrite à l'exemple 1 avec les conditions suivantes:

| | |
|---|---|
| Nature de l'oléfine | décène-1 |
| Température | 0°C |
| Cadence d'introduction de l'oléfine | 0,32 l/heure |
| Cadence d'introduction de l'acide | 0,88 l/heure |
| Rapport molaire acide/oléfine | 30 |
| Temps de contact | 20 minutes |

On obtient un produit fini défluoré dont la composition est la suivante:

| | % poids |
|---|---|
| Oléfine non oligomérisée | 2 |
| Dimère d'oléfine | 58 |
| Produits plus lourds | 40 |

*Exemple 5* (comparatif)

L'opération décrite à l'exemple 1 est réalisée avec un temps de contact long, dans les conditions suivantes:

| | |
|---|---|
| Nature de l'oléfine | tétradécène-1 |
| Température | 40°C |
| Cadence d'introduction de l'oléfine | 0,35 l/heure |
| Cadence d'introduction de l'acide | 0,25 l/heure |
| Rapport molaire acide/oléfine | 9 |
| Temps de contact | 40 minutes |

On obtient un produit fini défluoré dont la composition est la suivante:

| | % poids |
|---|---|
| Oléfine non oligomérisée | 20 |
| Dimère d'oléfine | 26 |
| Produits plus lourds | 54 |

### Exemple 6 (comparatif)

Une étape de mise en contact est réalisée comme à l'exemple 1 avec les conditions suivantes:

| | |
|---|---|
| Nature de l'oléfine | tétradécène-1 |
| Température | 30'C |
| Cadence d'introduction de l'oléfine | 1,4 l/heure |
| Cadence d'introduction de l'acide | 1 l/heure |
| Rapport molaire acide/oléfine | 9 |

Temps de contact dans le réacteur 10 minutes

Le milieu réactionnel ainsi obtenu est alors dirigé sur un décanteur de 4,5 litres où il séjourne pendant environ 2 heures.

La phase organique sortant de ce décanteur est portée durant un temps très bref (quelques secondes) à une température de 100 à 140°C de façon à éliminer la majeure partie de l'HF sous forme gazeuse.

Le milieu est ensuite soumis aux opérations de défluoration et de séparation décrites à l'exemple 1.

On obtient un produit fini défluoré dont la composition est la suivante:

| | % poids |
|---|---|
| Oléfine non oligomérisée | 25 |
| Dimère d'oléfine | 20 |
| Produits plus lourds | 55 |

### Exemple 7

On réalise une opération de mise en contact absolument identique à celle décrite à l'exemple 1.

Le mélange réactionnel qui sort en continu du réacteur dans lequel a eu lieu la mise en contact, est immédiatement recueilli dans une solution aqueuse à 20%, de façon à piéger quasi instantanément l'acide fluorhydrique sous forme de fluorure de potassium.

Ces opérations de défluoration et de séparation sont ensuite réalisées comme à l'exemple 1.

| | % poids |
|---|---|
| Oléfine $C_{14}$ non oligomérisée | 10 |
| Dimère d'oléfine $C_{14}$ | 67 |
| Produits plus lourds | 23 |

### Revendications

1. Procédé de dimérisation d'oléfines en $C_8$—$C_{20}$ par mise en contact desdites oléfines avec de l'acide fluorhydrique liquide à une température comprise entre —20 et 60°C, caractérisé:

en ce que ladite opération de mise en contact est réalisée pendant 1 à 30 minutes avec un rapport molaire acide fluorhydrique-oléfine compris entre 1/1 et 50/1;

en ce qu'après l'opération de mise en contact l'on sépare quasi instantanément du milieu de dimérisation l'acide fluorhydrique présent;

en ce que l'on sépare dudit milieu les composés fluorés par défluoration:

et en ce que l'on récupère les dimères d'oléfines obtenus.

2. Procédé selon la revendication 1, caractérisé en ce que l'opération de mise en contact est réalisée pendant 5 à 20 minutes, selon un rapport molaire acide fluorhydrique/oléfine compris entre 3/1 et 30/1.

3. Procédé selon la revendication 1 caractérisé en ce que l'opération de séparation quasi instantanée de l'acide fluorhydrique après l'opération de mise en contact, est réalisée par distillation rapide du type "flash".

4. Procédé selon la revendication 1 caractérisé en ce que l'opération de séparation quasi instantanée de l'acide fluorhydrique après l'opération de mise en contact, est réalisée par neutralisation du milieu de dimérisation par les agents classiques de neutralisation de l'acide fluorhydrique.

5. Procédé selon la revendication 4 caractérisé en ce que l'agent de neutralisation est de la potasse en solution aqueuse.

6. Procédé selon la revendication 1 ou la revendication 2 caractérisé en ce que l'opération de mise en contact est réalisée à une température comprise entre 0 et 40°C.

7. Procédé selon l'une quelconque des revendications 1 à 6 caractérisé en ce que l'oléfine mise en oeuvre est une α oléfine en $C_{10}$—$C_{16}$.

8. Dimères obtenus selon l'une quelconque des revendications 1 à 7.

### Patentansprüche

1. Verfahren zum Dimerisieren von $C_8$—$C_{20}$ Olefinen durch Zusammenbringen der Olefine mit flüssiger Fluorwasserstoffsäure bei einer Temperatur im Bereich von 20 bis 60°C, dadurch gekennzeichnet, dass

die Reaktionspartner während 1 bis 30 Minuten in einem Molverhältnis von Fluorwasserstoffsaüre zu Olefin zwischen 1:1 und 50:1 zusammengebracht werden.

dass man nach dem Zusammenbringen praktisch sofort die vorhandene Fluorwasserstoffsäure aus dem Dimerisationsmedium abtrennt,

dass man aus diesem Medium die fluorierten Verbindungen (mittels Entfluorierung) abtrennt,

und dass die erhaltenen Olefindimeren isoliert.

2. Verfahren nach Anspruch 1, dadurch gekennzeichnet, dass man die Reaktionspartner während 5 bis 20 Minuten bei einem Molverhältnis von Fluorwasserstoffsäure zu Olefin zwischen 3:1 und 30:1 zusammenbringt.

3. Verfahren nach Anspruch 1, dadurch gekennzeichnet, dass die praktisch sofortige Abtrennung der Fluorwasserstoffsäure nach dem Zusammenbringen der Reaktionspartner mittels Schnellverdampfung durchgeführt wird.

4. Verfahren nach Anspruch 1, dadurch gekennzeichnet, dass die praktisch sofortige Abtrennung der Fluorwasserstoffsäure nach dem Zusammenbringen der Reaktionspartner durch Neutralisation des Dimerisierungsmediums mit klassischen Neutralisierungsmitteln für die Fluorwasserstoffsaüre erfolgt.

5. Verfahren nach Anspruch 4, dadurch gekennzeichnet, dass das Neutralisierungsmittel eine wässrige Kalilauge ist.

6. Verfahren nach Anspruch 1 oder Anspruch 2, dadurch gekennzeichnet, dass man die Reaktionspartner bei einer Temperatur zwischen 0 und 40°C zusammenbringt.

7. Verfahren nach einem der Ansprüche 1 bis 6, dadurch gekennzeichnet, dass das eingesetzte Olefin ein $C_{10}$—$C_{16}$ $\alpha$-Olefin ist.

8. Dimere erhalten nach einem der Ansprüche 1 bis 7.

## Claims

1. Process for the dimerisation of $C_8$—$C_{20}$ olefines by bringing the said olefines into contact with liquid hydrofluoric acid at a temperature between —20 and 60°C, characterised in that the said operation for bringing the reactants into contact is carried out for 1 to 30 minutes with a molar ratio hydrofluoric acid/olefine of between 1/1 and 50/1, in that, after the operation for bringing the reactants into contact, the hydrofluoric acid present is separated from the dimerisation medium virtually instantaneously, in that the fluorinated compounds are separated from the said medium by defluorination, and in that the olefine dimers obtained are recovered.

2. Process according to Claim 1, characterised in that the operation for bringing the reactants into contact is carried out for 5 to 20 minutes with a molar ratio hydrofluoric acid/olefine of between 3/1 and 30/1.

3. Process according to Claim 1, characterised in that the operation for separating off the hydrofluoric acid virtually instantaneously, after the operation for bringing the reactants into contact, is carried out by means of rapid distillation of the "flash" type.

4. Process according to Claim 1, characterised in that the operation for separating off the hydrofluoric acid virtually instantaneously, after the operation for bringing the reactants into contact, is carried out by neutralising the dimerisation medium with the conventional agents for neutralising hydrofluoric acid.

5. Process according to Claim 4, characterised in that the neutralisation agent is an aqueous solution of potassium hydroxide.

6. Process according to Claim 1 or Claim 2, characterised in that the operation for bringing the reactants into contact is carried out at a temperature between 0 and 40°C.

7. Process according to any one of Claims 1 to 6, characterised in that the olefine used is a $C_{10}$—$C_{16}$ $\alpha$-olefine.

8. Dimers obtained according to any one of Claims 1 to 7.